# EUROPÄISCHE PATENTSCHRIFT

(11) **EP 2 052 232 B1**
(45) Veröffentlichungstag und Bekanntmachung des Hinweises auf die Patenterteilung: **07.08.2013**
(21) Anmeldenummer: 07803476.6
(22) Anmeldetag: 13.09.2007
(51) Int. Cl.: G01N 33/82, G01N 33/92, G01N 1/40

(54) **BESTIMMUNG VON INHALTSSTOFFEN AUS BIOLOGISCHEN MATERIALIEN**
DETERMINATION OF BIOLOGICAL MATERIAL INGREDIENTS
DÉTERMINATION DE SUBSTANCES CONTENUES DANS DES MATÉRIAUX BIOLOGIQUES

(30) Priorität: 13.09.2006 DE 102006044795
(43) Veröffentlichungstag der Anmeldung: 29.04.2009
(73) Patentinhaber: BaPat GmbH, 14513 Teltow (DE)
(72) Erfinder: Schweigert, Florian, 12205 Berlin (DE)
(74) Vertreter: Wablat, Wolfgang
(86) Internationale Anmeldenummer: PCT/EP2007/059664
(87) Internationale Veröffentlichungsnummer: WO 2008/031874

(56) Entgegenhaltungen:
- EP-A- 1 277 760
- WO-A-01/01116
- WO-A-01/01117
- WO-A-01/77675
- WO-A-02/061096
- US-A- 4 282 001
- US-A- 5 994 141
- SCHWEIGERT F J ET AL: "Improved extraction procedure for carotenoids from human milk" INTERNATIONAL JOURNAL FOR VITAMIN & NUTRITION RESEARCH, HOGREFE AND HUBER, BERNE, CH, Bd. 70, Nr. 3, Mai 2000 (2000-05), Seiten 79-83, XP008085463 ISSN: 0300-9831
- SCHWEIGERT F J ET AL: "Effect of age on the levels of retinol and retinyl esters in blood plasma, liver and kidney of dogs" INTERNATIONAL JOURNAL FOR VITAMIN & NUTRITION RESEARCH, HOGREFE AND HUBER, BERNE, CH, Bd. 68, Nr. 4, 1998, Seiten 237-241, XP008085465 ISSN: 0300-9831
- "Varian, Inc. Cary 100/300 UV-Vis spectrophotometers" INTERNET ARTICLE - PRODUCT INFORMATION, [Online] August 2005 (2005-08), XP002457655 Gefunden im Internet: URL:http://www.varianinc.com/image/vimage/ docs/products/spectr/uv/brochure/si-0135.p df> [gefunden am 2007-11-05]
- MÜLLER H: "Anwendung von Ethoxyquin zur Qualitätserhaltung pflanzlicher Produkte." 1980, FETTE SEIFEN ANSTRICHMITTEL 1980 BUNDESFORSCHUNGSANSTALT FÜR ERNÄHRUNG, ENGESSERSTRASSE 20, 7500 KARLSRUHE 1, FEDERAL REPUBLIC OF GERMANY, VOL. 82, NR. 5, PAGE(S) 200 , XP002457657 das ganze Dokument
- "Starna Cells, Inc. Catalog of: Spectrophotometer Cells, Fluorometer Cells, Colorimeter Cells, Laser cells" INTERNET ARTICLE - PRODUCT INFORMATION, [Online] August 2002 (2002-08), XP002457656 Gefunden im Internet: URL:http://web.archive.org/web/20030505190 309/http://www.starnacells.com/d_download/ Starna.pdf> [gefunden am 2007-11-05]
- PETERSON J E ET AL: "Simplified extraction and cleanup for determining organochlorine pesticides in small biological samples", BULLETIN OF ENVIRONMENTAL CONTAMINATION AND TOXICOLOGY, SPRINGER VERLAG, NEW YORK, NY, US, vol. 15, no. 2, 1 February 1976 (1976-02-01), pages 135-139, XP008142495, ISSN: 0007-4861
- N RUIZ-LÓPEZ: 'Sequential one-step extraction and analysis of triacylglycerols and fatty acids in plant tissues' ANALYTICAL BIOCHEMISTRY Bd. 317, Nr. 2, 15 Juni 2003, Seiten 247 - 254, XP055007375 DOI: 10.1016/S0003-2697(03)00139-8 ISSN: 0003-2697
- GARCES R ET AL: "One-Step Lipid Extraction and Fatty Acid Methyl Esters Preparation from Fresh Plant Tissues", ANALYTICAL BIOCHEMISTRY, ACADEMIC PRESS INC, NEW YORK, vol. 211, no. 1, 15 May 1993 (1993-05-15), pages 139-143, XP024763895, ISSN: 0003-2697, DOI: 10.1006/ABIO.1993.1244 [retrieved on 1993-05-15]
- SHAHIDI AND P K J P D WANASUNDARA F: "Chapter 5: Extraction and Analysis of Lipids", FOOD LIPIDS: CHEMISTRY, NUTRITION, AND BIOTECHNOLOGY, SECOND EDITION,, 1 January 2002 (2002-01-01), pages 133-168, XP008142572, ISBN: 0-8247-4448-9
- O'CONNOR ET AL: "Candida cylindracea lipase -catalyzed synthesis of retinyl and oleyl palmitates;carbon chain length dependence of esterase activity", AUSTRALIAN JOURNAL OF CHEMISTRY, CSIRO, AU, vol. 45, 22 June 1992 (1992-06-22), pages 641-649, XP002013342, ISSN: 0004-9425
- KYUNG-JIN YEUM ET AL: 'Human plasma carotenoid response to the ingestion of controlled diets high in fruits and vegetabIes' AMERICAN JOURNAL OF CLINICAL NUTRITION Bd. 64, Nr. 4, 01 Oktober 1996, Seiten 594 - 602, XP055007380 ISSN: 0002-9165
- JULIAN MCCLEMENT: '5. Analysis of lipids' 30 März 2004, Seiten 1 - 14, XP055007361 Gefunden im Internet: <URL:http://people.umass.edu/~mcclemen/581L ipids.html> [gefunden am 2011-09-16]
- NICHOLS JORDAN ET AL: "Principles of lipid analysis - extraction of lipids from foods, Chapter 3", CHEMICAL, BIOLOGICAL, AND FUNCTIONAL ASPECTS OF FOOD LIPIDS / ED. BY ZDZIS AW E. SIKORSKI; ANNA KO AKOWSKA, BOCA RATON, FLA. [U.A.] : CRC PRESS, 2011, US, vol. Second edition, 1 October 2010 (2010-10-01), pages 36-42, XP008142479, ISBN: 978-1-4398-0237-3
- DAVID S NICHOLS ED - ZDZIS AW E SIKORSKI ET AL: "Chapter 9: Principles of lipid analysis 9.2 Extraction of lipids from foods", 1 January 2002 (2002-01-01), CHEMICAL AND FUNCTIONAL PROPERTIES OF FOOD LIPIDS, CRC PRESS, USA, PAGE(S) 167 - 188, XP008157760, ISBN: 978-1-58716-105-6
- VINCENT G NEALIS ET AL: "Seasonal Changes in Foliar Terpenes Indicate Suitability Of Douglas-fir Buds for Western Spruce Budworm", JOURNAL OF CHEMICAL ECOLOGY, KLUWER ACADEMIC PUBLISHERS-PLENUM PUBLISHERS, NE, vol. 31, no. 4, 1 April 2005 (2005-04-01), pages 683-696, XP019370470, ISSN: 1573-1561, DOI: 10.1007/S10886-005-3538-8

## Beschreibung

Die vorliegende Erfindung betrifft ein. Verfahren zur Analyse von Inhaltsstoffen, insbesondere von Lipiden und/oder Vitaminen, biologischer Materialien mit einer Anreicherung der Inhaltsstoffe aus den biologischen Materialien, bei welchem den biologischen Materialien, welche sich in einem für spektroskopische Untersuchungen geeigneten Gefäß mit einer hydrophoben Oberfläche befinden in einem einzigen Schritt ein Lösungsmittelgemisch zugegeben wird, welches polare organische Lösungsmittel und unpolare organische Lösungsmittel umfasst, welches die Inhaltsstoffe extrahiert, wobei die biologischen Materialien bei der Extraktion in eine verfestigte Form überführt werden, wobei eine Verfestigung eine Viskositätszunahme der biologischen Materialien bedeutet, so dass ein verfestigtes Sediment und eine flüssige organische Phase als Überstand rein aufgrund von Gravitationskräften durch zu Boden sinken der biologischen Materialien ohne Zentrifugation gebildet werden, und die in den Überstand extrahierten Inhaltsstoffe im selben Gefäß direkt spektrophotometrisch oder fluorimetrisch untersucht werden.

Moderne Verfahren zur Analyse von biologischen Materialien weisen oftmals Schritte zur Abtrennung, Extraktion, Isolation und/oder Anreicherung von Bestandteilen sowie Inhaltsstoffen der biologischen Materialien auf. Solche Verfahrensschritte sind sowohl in der qualitativen als auch quantitativen Analytik zur Abtrennung von störenden oder ergebnisverfälschenden Substanzen unumgänglich. Ferner setzen sich sogenannte Hochdurchsatzverfahren oder mikrofluidische Systeme im analytischen Bereich durch, welche ihre gesamte Funktionalität auf Chipkartengröße verkleinert bereitstellen. Solche Verfahren oder Systeme werden umgangssprachlich unter Begriffen, wie Westentaschenlabor, Chiplabor ("lab-on-a-chip") oder "point-of-care"-systems (diagnostische Systeme, welche direkt vor Ort an Patienten eingesetzt werden können) subsummiert. Für eine solche Art der Analytik ist eine weitgehende Miniaturisierung erforderlich. Die Miniaturisierung solcher Systeme birgt eine Anfälligkeit gegenüber Verunreinigungen in sich, welche Oberflächen sowie Kapillarsysteme blockieren und als Folge unbrauchbar machen können. Daher benötigen solche Systeme oder Verfahren bis zur endgültigen Analytik häufig eine arbeitsintensive Probenvorbereitung mit zeit- und geräteintensiven Zwischenschritten. Solch eine Problematik trifft insbesondere die Analytik von Blut oder Lebensmitteln. In human- als auch veterinärmedizinischen Laboren werden als häufigste Untersuchungen Analysen von Blut zur Diagnostik vorgenommen. Üblicherweise werden die Untersuchungen am zellfreien Anteil von Vollblutproben durchgeführt, insbesondere am Blutserum oder Blutplasma. Untersuchungen an Vollblut werden aufgrund einer Lysegefahr von Blutzellen, insbesondere einer Hämolysegefahr von Erythrozyten, ungern vorgenommen. Als konventionelle Techniken zur Abtrennung zellulärer Blutbestandteile kommen vor der Analytik Zentrifugation und/oder Filtration in Frage. Die Abtrennung solcher korpuskulärer Bestandteile dient dazu, mögliche Störeffekte sowohl von Zellen als auch von Zellfragmenten sowie von Lysisprodukten, insbesondere bei der Chiplabor-Analytik, zu vermeiden. Üblicherweise erfolgt zur Zeit die Abtrennung auch bei der Chiplabor-Analytik konventionell mittels Zentrifugations- oder Filtrationsschritten. Zur direkten Abtrennung der Zellen wird eine Filtration auf den Chiplaboren bevorzugt. Dabei zeigt sich als Hauptproblem ein rasches Verstopfen von Kapillaren, unabhängig vom jeweils verwendeten Filtersystem. Aufgrunddessen können als Grenzmenge meist nur Volumina von wenigen Mikrolitern aufgetragen werden. Ähnliche Probleme betreffen auch die Analytik von Lebensmitteln.

Die notwendige Abtrennung durch Zentrifugation oder Filtration führt zu einem erhöhten Zeitaufwand sowie einem erhöhten Bedarf an Reagenzien, Geräten und biologischen Materialien, insbesondere Blut, für die Analytik. Es besteht somit ein großer Bedarf an Verfahren zur Analyse von Inhaltsstoffen biologischer Materialien, insbesondere Blut, welche mit unfiltrierten oder unzentrifugierten Proben durchgeführt werden können.

Den Stand der Technik bilden mehrschrittige Verfahren, wie sie beispielsweise in der WO 01/01117A1 und der WO 01/01116 A1 beschrieben sind. Beide Schriften unterscheiden sich nur darin, dass die erste eine qualitative UV-Auswertung der Proben verwendet, wohingegen die zweite eine semi-quantitative fluorometrische Auswertung nutzt. Die Hauptausführungsform ist ein mehrschrittiges Verfahren, bei welchem biologische Materialen in einem ersten Schritt mit einem organischen Lösungsmittel, welches vorzugsweise polarer Natur ist, versetzt werden. Die resultierende flüssige Phase (der Extrakt) wird abgenommen und anschließend in einem zweiten Schritt mit einer Mischung aus Wasser, Salz und einem nicht-mit-Wasser-mischbaren organischen Lösungsmittel versetzt. Der daraus resultierende Extrakt wird abgenommen und anschließend untersucht. Nachteilig hierbei ist, dass mehrere Schritte erforderlich sind. Vereinfachungen dieses Verfahrens sind, dass entweder das polare Lösungsmittel aus dem ersten Schritt weggelassen und in einem ersten Schritt mit dem nicht-mit-Wasser-mischbaren Lösungsmittel behandelt und anschließend in einem zweiten Schritt mit der Mischung aus Wasser und Salz behandelt wird, oder es wird in einem einschrittigen Verfahren direkt mit einer Mischung aus nicht-mit-Wassermischbarem Lösungsmittel, Wasser und Salz behandelt. Ein relevanter Nachteil ist, dass die Gegenwart von Salz (und Wasser) in allen Fällen essentiell ist. Diese Komponenten sind notwendig, um die Emulsionen zwischen dem mit-Wasser-mischbaren und dem nicht-mit-Wasser-mischbaren Lösungsmittel aufzubrechen.

### Aufgabe und Lösung

Es ist daher Aufgabe der vorliegenden Erfindung, ein einschrittiges Verfahren zur Analyse von Inhaltsstoffen biologischer Materialien bereitzustellen, welches auf eine Zentrifugation oder Filtration von Proben der biologischen Materialien vor ihrer Analytik verzichtet.

Die vorliegende Erfindung löst das ihr zugrundeliegende technische Problem durch das eingangs genannte. Verfahren zur Analyse von Inhaltsstoffen, insbesondere von Lipiden und/oder Vitaminen, biologischer Materialien mit einer Anreicherung der Inhaltsstoffe aus den biologischen Materialien, bei welchem den biologischen Materialien, welche sich in einem für spektroskopische Untersuchungen geeigneten Gefäß mit einer hydrophoben Oberfläche befinden in einem einzigen Schritt ein Lösungsmittelgemisch zugegeben wird, welches polare organische Lösungsmittel und unpolare organische Lösungsmittel umfasst, welches die Inhaltsstoffe extrahiert, wobei die biologischen Materialien bei der Extraktion in eine verfestigte Form überführt werden, wobei eine Verfestigung eine Viskositätszunahme der biologischen Materialien bedeutet, so dass ein verfestigtes Sediment und eine flüssige organische Phase als Überstand rein aufgrund von Gravitationskräften durch zu Boden sinken der biologischen Materialien ohne Zentrifugation gebildet werden, und die in den Überstand extrahierten Inhaltsstoffe im selben Gefäß direkt spektrophotometrisch oder fluorimetrisch untersucht werden. Im Rahmen der hier vorliegenden Erfindung sollen unter "biologischen Materialien" von Pflanzen, Tieren, Menschen und/oder Mikroorganismen gewonnene Materialien, insbesondere körpereigene Materialien, vorzugsweise Proben von körpereigenen Materialien, verstanden werden.

Unter "Mikroorganismen" sollen im Rahmen der hier vorliegenden Erfindung neben beispielsweise Eukaryoten, wie Algen, Prokaryoten und/oder Pilzen, wie Hefen, auch Viren umfasst sein. Insbesondere sollen "biologische Materialien" aus Kultur gewonnene Organismen sowie Kulturüberstände umfassen.

Zur Bereitstellung der biologischen Materialien werden im erfindungsgemäßen Verfahren Proben der biologischen Materialien eingesetzt, bevorzugt von Menschen oder tierischen Organismen entnommene Proben. Zweckmäßigerweise können auch abgegebene bzw. sezernierte biologische Materialien verwendet werden. Weiterhin ist es auch möglich, die biologischen Materialien vor ihrer Verwendung im erfindungsgemäßen Verfahren zu kultivieren, insbesondere außerhalb vom menschlichen oder tierischen Körper.

Unter einer "Verfestigung" soll im Rahmen der hier vorliegenden Erfindung eine Viskositätszunahmel der biologischen Materialien verstanden werden, so dass ein verfestigtes Sediment gebildet wird. Ferner kann auch eine Viskosität erreicht werden, welche zwischen der einer Flüssigkeit und der eines Feststoffes liegt. Die Verfestigung kann beispielsweise durch ein Ausfällen erreicht werden.

Beschrieben werden als verfestigte Form oder Verfestigung Gele oder gelartige Zustände. Aus technischer Sicht ist es relativ einfach die Gele oder gelartige Zustände zu beschreiben. Eine allgemein akzeptierte umfassende Definition für die Gele wird allerdings noch gesucht.

Die Gele werden üblicherweise als feindisperse (kolloidale) Systeme aus mindestens einer festen Phase und einer flüssigen Phase beschrieben. In manchen Gelen kann die feste Phase in Form eines schwammartigen, dreidimensionalen Netzwerkes vorliegen, in welchem die flüssige Phase aufgenommen ist. Die Gele befinden sich hinsichtlich ihrer mechanischen Eigenschaften in einem fest-ähnlichen (solid-like) Zustand. Ein solcher Zustand zeichnet sich durch gleichmäßig im gesamten System verteilte Komponenten aus. Die Gele können sowohl durch gleichmäßige Strukturen, insbesondere geordnete Strukturen, als auch durch ungeordnete Strukturen, insbesondere im Falle von Netzwerken aggregierter Polymere, gekennzeichnet sein. Ferner können die Gele nach physikalischer und chemischer Art ihrer Bindungskräften als auch ihrer flüssigen Phasen charakterisiert werden. Handelt es sich bei der flüssigen Phase um wäßrige Medien, wird das Gel als "Hydrogel" bezeichnet. Im Falle von organischen Medien wird das Gel als "Organogel" bezeichnet. Als "Amphiphilogel" werden Gele bezeichnet, welche oberflächenaktive Substanzen als flüssige Phase aufweisen.

Üblicherweise entsteht ein Gel durch eine Lösung eines Gelators in einem erwärmten Lösungsmittel. Durch Abkühlen beginnt die Lösung zu gelieren. Die Gelbildung beginnt, wenn sich die Löslichkeit des Gelators bzw. Geliermittels im Lösungsmittel reduziert. Dies führt zu einer Zusammenlagerung von Gelatormolekülen (Selbstaggregation). Teile des Lösungsmittels werden in dem Gelator gebunden. Als Ergebnis verfestigt sich das Lösungsmittel in seinem Volumen, insbesondere seinem ganzen Volumen, und es kann sein eigenes Gewicht tragen.

Als Gelatoren oder Geliermittel sind je nach Art der Gele verschiedene niedermolekulare Substanzen bekannt. In der Natur finden sich zahlreiche Moleküle, welche in wäßrigen Lösungen die Hydrogele bilden. Geliermittel, welche insbesondere in Lebensmitteln als Zusatzstoffe Wasser binden bzw. im Wasser quellen, d. h. zu einer Gelierung führen, sind zum Beispiel Agar-Agar, Alginsäure, Carrageen, Gelatine, Guarkernmehl, Gummi arabicum, Johannisbrotkernmehl, Pektin, insbesondere amidiertes Pektin, sowie modifizierte Starke.

Als Gelatoren für Organogele sind beispielsweise Fettsäuren, Fettsäurederivate, insbesondere Metallsalze von Fettsäuren, Steroidderivate, insbesondere Cholesterinderivate, aminosäurehaltige Gelatoren, aromatische Gelatoren, insbesondere Anthrachinonderivate, beispielsweise Antrylderivate, Calixarene, Pyridine, als auch Sorbitol- und Polyolderivate zu nennen. Ein bekanntes Organogel wird durch Lecithin gebildet. Für die Verfestigung der biologischen Proben sind neben der Gelbildung auch weitere Techniken dem Fachmann bekannte.

Das Verfahren gemäß der vorliegenden Erfindung ist zur Analyse von Inhaltsstoffen, vorzugsweise von Lipiden und/oder Vitaminen Techniken, biologischer Materialien vorgesehen. Insbesondere dient das Verfahren einer Extraktion von lipophilen Inhaltsstoffen. Den biologischen Materialien werden in einem einzigen Schritt die eingangs genannten Lösungsmittelgemische zugegeben, in welche die Inhaltsstoffe während der Extraktion überführt werden. Die biologischen Materialien liegen bevorzugt im wäßrigen Milieu vor.

Überraschenderweise werden die biologischen Materialien während der Extraktion nach dem erfindungsgemäßen Verfahren in eine verfestigte Form überführt. Durch eine Behandlung mit den eingangs genannten Lösungsmittelgemischen wird ein mehrphasiges System, insbesondere ein aus zwei Phasen bestehendes System, gebildet. Während der voranschreitenden Verfestigung, vorzugsweise gleichzeitig, werden die biologischen Materialien, insbesondere Proben der biologischen Materialien, extrahiert.

Die Behandlung der biologischen Materialien mit den Lösungsmittelgemischen kann so erfolgen, dass die biologischen Materialien zu den Lösungsmittelgemischen gegeben werden, es können aber auch die Lösungsmittelgemische zu den biologischen Materialien gegeben werden.

Durch die Verfestigung der biologischen Materialien werden die für die Extraktion vorgesehenen Lösungsmittel von den biologischen Materialien abgetrennt. Es wird ein verfestigtes Sediment und ein Überstand gebildet. Der Überstand umfaßt die zur Extraktion vorgesehenen Lösungsmittel und die Inhaltsstoffe, insbesondere lipophile Inhaltsstoffe, welche in die organischen Lösungsmittel überführt worden sind. Vorteilhafterweise werden somit die biologischen Materialien durch die verfestigte Form von den extrahierten im Überstand befindlichen Inhaltsstoffen separiert. Die biologischen Materialien bilden das verfestigte Sediment, die Inhaltsstoffe befinden sich im organischen Lösungsmittelgemisch darüber und können im Weiteren durch bekannte Analysetechniken untersucht werden.

### Möglicher Wirkungsmechanismus

Durch ein Mischen der biologischen Materialien, beispielsweise Blut, mit den im erfindungsgemäßen Verfahren eingesetzten organischen Lösungsmittelgemischen von polaren organischen Lösungsmitteln und unpolaren organischen Lösungsmitteln, kommt es zu einer Phasentrennung. Das polare organische Lösungsmittel geht entsprechend seines Verteilungskoeffizenten im wesentlichen in das biologische Material, welches eine wässrige Phase darstellt. Da in der wässrigen Phase Inhaltsstoffe, vorzugsweise lipophile Inhaltsstoffe, beispielsweise in Form von Membranbestandteilen und Lipoproteinen vorhanden sind, reichert sich das polare organische Lösungsmittel in diesen Strukturen an. Die polaren organischen Lösungsmittel können in Zellmembranen, wie beispielsweise die Zellmembran der Erythrozyten im Vollblut, eindringen und mit dem Lipidanteil interagieren. Es kommt zu zwei Effekten: Stark lipophile Inhaltsstoffe werden aus ihrem Verbund gelöst und können in den Überstand extrahiert werden. Mit geringer lipophilen Inhaltsstoffen in den Zellmembranen oder lipidhaltigen Strukturen interagiert das polare organische Lösungsmittel. Geeignete Lipide für die zur Viskositätszunahme führenden Effekte sind die in Zellwänden und Lipoproteinen vorkommenden Phospholipide. Biologische Membranen weisen eine Vielzahl von Phospholipiden unterschiedlicher chemischer Zusammensetzung auf.

Eine Interaktion mit den Phospholipiden als auch eine Entfernung von Cholesterin aus der Zellmembran führt zu einer Vernetzung der Zellmembranen (Interdigitation) und zu der Viskositätszunahme. Eine Anwesenheit von Cholesterin verstärkt ein fluides Verhalten (Fluidität) der Zellmembran durch eine Bildung einer sogenannten Lₒ-Phase ("liquid-orderd") und verhindert die Vernetzung. Mit steigendem Cholesterinanteil in Modellmembranen kommt es zu einer Reduktion der Membranvernetzung durch polare organische Lösungsmittel wie Alkohole (Methanol, Ethanol, Propanol, Butanol). Zum erreichen vergleichbarer Effekte sind bei einer Cholesterinanreicherung höhere Alkoholkonzentrationen notwendig. Auch oberflächenaktive Substanzen in sublytischen Konzentrationen und verschiedene kurzkettige Alkohole führen zu einer Depletion von Cholesterin aus Membranen. Die Möglichkeit der Interaktion von Alkoholen mit den membranständigen Phospholipiden wird auch durch Bebachtungen gestützt, dass Alkohole eher Cholesterin als Phospholipide extrahieren. Generell lassen sich Phospholipide schlechter extrahieren.

Vorteilhafterweise werden durch das erfindungsgemäße Verfahren klare Lösungen als Überstände gebildet, welche einer weiteren Analytik ohne weitere Behandlungen zugeführt werden können. Bevorzugterweise werden Lösungsmittelgemische verwendet, welche eine geringere spezifische Dichte als Wasser aufweisen. Somit ist sichergestellt, dass sich ein flüssiger Überstand bildet. Daneben kann es zweckmäßig sein, Lösungsmittel mit einer höheren Dichte als Wasser zu verwenden, um ein Aufrahmen der verfestigten biologischen Materialien zu erreichen. Dies kann, insbesondere je nach verwendetem Probengefäß, vorteilhaft für weitere spektroskopische Untersuchungen sein. Im erfindungsgemäßen Verfahren werden bevorzugt Lösungsmittel mit einer geringeren spezifischen Dichte als Wasser eingesetzt.

Vorzugsweise werden im erfindungsgemäßen Verfahren biologische Materialien verwendet, welche die gewünschte Verfestigung während einer Durchführung des Verfahrens aufweisen.

In einer weiteren Ausführungsform umfassen die biologischen Materialien pflanzliche, tierische, menschliche und/oder mikrobielle Materialien, welche insbesondere aus Zellkulturen stammen.

Erfindungsgemäß können alle biologischen Materialien verwendet werden, welche der Extraktion zugeführt werden können.

In einer weiteren bevorzugten Ausführungsform des Verfahrens werden die biologischen Materialien vor ihrem Einsatz Vorbehandlungen, insbesondere Reinigungsvorgängen, unterworfen.

Als Vorbehandlungen kommen beispielsweise Aufschlüsse in Betracht. Als Aufschlüsse kommen Behandlungen mit Homogenisatoren in Frage. Als Homogenisatoren sind beispielsweise Cutter, Ultraturrax-Geräte, Mühlen usw. zu nennen. Die biologischen Materialien werden hierdurch einer Zerkleinerung unterworfen, um ihre Oberfläche zu vergrößern. Durch die Zerkleinerung wird die Extraktion in ihrer Effizienz gesteigert. Daneben kommen auch Behandlungen mit Kälte, insbesondere mit flüssigem Stickstoff, in Frage. Die Aufschlüsse dienen dazu, Inhaltsstoffe aus den biologischen Materialien freizusetzen, und somit einer Extraktion zugängig zu machen. Die biologischen Materialien können als Vorbehandlung auch einer Abtrennung von Verunreinigungen und/oder störenden Substanzen, insbesondere Waschvorgängen, beispielsweise mit Pufferlösungen, unterworfen werden.

In einer weiteren bevorzugten Ausführungsform des Verfahrens werden als biologische Materialien Gewebe, Organe und/oder Körperflüssigkeiten verwendet.

Bevorzugterweise werden solche biologischen Materialien verwendet, welche im medizinischen Bereich für eine Diagnostik und/oder Therapieverfolgung eingesetzt werden.

In einer weiteren bevorzugten Ausführungsform des Verfahrens werden als Körperflüssigkeiten Blut, Plasma, Serum, Harn, Amnionflüssigkeit, Uterusflüssigkeit, Folikelflüssigkeit, Synovia, Sperma, Lungenflüssigkeit und/oder Sekrete verwendet.

Bevorzugterweise handelt es sich bei den Sekreten um Milch, Schweiß, Tränenflüssigkeit, Speichel und/oder Magen-Darm-Trakt-Sekrete, insbesondere um Gallenflüssigkeiten und/oder Pankreassekret. Erfindungsgemäß kann als Körperflüssigkeit Blut, vorzugsweise Vollblut, verwendet werden. Bevorzugterweise wird als biologisches Material im erfindungsgemäßen Verfahren Blut verwendet. Als Vorbehandlung wird das Blut bevorzugterweise mit gerinnungshemmenden Stoffen behandelt, insbesondere mit polyanionischen Polysacchariden, vorzugsweise mit Heparin und/oder Heparinoiden. Weiterhin können als Antikoagulanzien AntiThrombin III, insbesondere in Form von Heparin-Anti-Thrombin-Komplexen, eingesetzt werden. Weiterhin können auch körperfremde Antikoagulanzien eingesetzt werden. Als körperfremde Antikoagulanzien kommen insbesondere Vitamin-K-Antagonisten oder KalziumKomplexbildner in Frage. Als Vitamin-K-Antagonisten sind insbesondere Cumarine zu nennen, als Kalziumkomplexbildner sind insbesondere Citrat, Oxalat, vorzugsweise Ethylendiamintetraacetat (EDTA), zu nennen.

Vorteilhafterweise wird durch das erfindungsgemäße Verfahren eine Hämolyse des Blutes, insbesondere der Blutzellen, vermieden. Durch die erfindungsgemäße Verfestigung des Blutes werden die Blutzellen in einer gelartigen Umgebung eingebettet und somit vor einer Hämolyse geschützt.

Vorteilhaft ist ferner, dass die Extraktion manuell durch Schütteln, insbesondere vorsichtiges, eine Hämolyse vermeidendes Schütteln, durchgeführt wird. Für die Extraktion des Blutes mit den Lösungsmitteln können auch Hilfsmittel, insbesondere Rütteltische, Schwenkwippen, Überkopfschüttler, Magnetrührer und andere Rührtechniken eingesetzt werden. Ein manuelles Mischen hat den Vorteil, von einer Stromquelle oder von elektrischen Geräten unabhängig die Extraktion vornehmen zu können.

In einer weiteren bevorzugten Ausführungsform des Verfahrens werden als biologische Materialien Lebensmittel tierischen und/oder pflanzlichen Ursprungs, insbesondere Homogenisate von Lebensmitteln, eingesetzt.

Als Lebensmittel kommen insbesondere Eier, vorzugsweise Volleigelb, Obst, Gemüse, insbesondere Karotten, und/oder Fisch und/oder Fleisch in Frage. Daneben können mit dem erfindungsgemäßen Verfahren als Lebensmittel Säfte, insbesondere Frucht- und/oder Gemüsesäfte, vorzugsweise Karottensäfte, untersucht werden.

Bevorzugterweise kommen für das erfindungsgemäße Verfahren Lebensmittel in Betracht, welche für die Ernährung von Menschen essentielle Inhaltsstoffe, vorzugsweise lipophile Inhaltsstoffe, aufweisen.

In einer weiteren bevorzugten Ausführungsform des Verfahrens werden als polares organisches Lösungsmittel polar protische Lösungsmittel verwendet, insbesondere Alkohole.

In einer weiteren bevorzugten Ausführungsform des Verfahrens sind die polar protischen Lösungsmittel ausgewählt aus der Gruppe, umfassend

Methanol, Ethanol, 1-Propanol, 2-Propanol (Isopropanol), Butanol, Pentanol, Hexanol sowie Gemische davon.

Überraschenderweise bewirkt der Einsatz von Alkoholen im erfindungsgemäßen Verfahren die Verfestigung der biologischen Proben. Bevorzugt werden Gemische von Ethanol und 2-Propanol (Isopropanol) verwendet.

In einer weiteren bevorzugten Ausführungsform des Verfahrens wird als polares organisches Lösungsmittel mindestens ein polar aprotisches Lösungsmittel verwendet, insbesondere werden Ester, vorzugsweise Essigsäureethylester, verwendet.

Vorteilhafterweise lassen sich im erfindungsgemäßen Verfahren neben den bevorzugten polar protischen Lösungsmitteln auch polar aprotische Lösungsmittel als polares organisches Lösungsmittel einsetzen.

In einer weiteren Ausführungsform des Verfahrens werden als polar aprotische Lösungsmittel Nitrile, vorzugsweise Acetonitril, verwendet.

In einer weiteren Ausführungsform des Verfahrens werden als polar aprotische Lösungsmittel Ketone, vorzugsweise Aceton, verwendet. In einer weiteren Ausführungsform des Verfahrens werden als polar aprotische Lösungsmittel Dimethylsulfoxid und/oder N,N-Dimethylformamid verwendet.

In einer weiteren Ausführungsform des Verfahrens werden als polar aprotische Lösungsmittel Ether, insbesondere Diethylether, verwendet.

Die bisher genannten Lösungsmittel dienen der Verfestigung der biologischen Materialien. Bevorzugterweise werden die polaren Lösungsmittel abhängig von den biologischen Materialien in Form von Gemischen eingesetzt.

Als unpolare organische Lösungsmittel, werden insbesondere Alkane, vorzugsweise C5- bis C12-Alkane, verwendet.

In einer weiteren bevorzugten Ausführungsform des Verfahrens werden als unpolare organische Lösungsmittel Hexan, Heptan und/oder Octan, insbesondere Isooctan, verwendet.

In einer weiteren Ausführungsform des Verfahrens werden als unpolare organische Lösungsmittel Aromaten, insbesondere Toluol und/oder Benzol, verwendet.

Die genannten unpolaren Lösungsmittel dienen im erfindungsgemäßen Verfahren, d.h. im erfindungsgemäßen Lösungsmittelgemisch, als Extraktionsmittel für die Inhaltsstoffe, insbesondere für die lipophilen Inhaltsstoffe.

Erfindungsgemäß kann es zweckmäßig sein, Derivate und oder Isomere von organischen Lösungsmittelmolekülen zu verwenden. Die Lösungsmittel können wasserfrei, wasserhaltig, verzweigt, unverzweigt, cyclisch, acyclisch, halogeniert oder unhalogeniert sein.

Eine Einteilung in polare oder unpolare Lösungsmittel kann in der Technik unter verschiedenen Gesichtspunkten geschehen. Beispielsweise können aus der Chemie bekannte Definitionen von Polarität oder Lösungsmittelverhalten angewendet werden.

Daneben findet in der Praxis ein Polaritätsindex nach Snyder oder Keller Verwendung (Snyder, Principles of absorption chromatography, Decker, New York, 1968; Keller, Analytical chemistry, Weinheim, 1998, Seite 195), um Lösungsmittel oder Lösungsmittelgemische zu klassifizieren. Danach wird unter polaren Lösungsmitteln oder Lösungsmittelgemischen ein Lösungsmittel oder Lösungsmittelgemisch mit einem Polaritätsindex von 4 bis 8, insbesondere von 5 bis 7, vorzugsweise von 5,5 bis 6,5, nach Snyder verstanden. Nicht erfindungsgemäße polare Lösungsmittel sind beispielsweise Wasser, insbesondere wäßrige Lösungen. Polar aprotische Lösungsmittel sind beispielsweise Aceton, Acetonitril, Essigsäureethylester, Dimethylsulfoxid oder N,N-Dimethylformamid. Polar protische Lösungsmittel sind beispielsweise Alkohole, welche einen Alkylrest mit 1 bis 6 Kohlenstoffatomen aufweisen, beispielsweise Methanol, Ethanol, 1-Propanol, 2-Propanol (Isopropanol), Buthanol, Pentanol oder Hexanol.

Unter einem unpolaren Lösungsmittel oder unpolaren Lösungsmittelgemisch wird ein Lösungsmittel oder Lösungsmittelgemisch verstanden, welches im Vergleich zu einem Referenzlösungsmittel oder Referenzlösungsmittelgemisch einen um 0,3 oder mehr kleineren Polaritätsindex aufweist. Bevorzugt ist ein um 0,5 kleinerer Polaritätsindex, insbesondere ein um 1 kleinerer Polaritätsindex, vorzugsweise ein um mehr als 2 kleinerer Polaritätsindex. Folglich weist der Polaritätsindex des unpolaren Lösungsmittels oder Lösungsmittelgemischs einen Wert von 5 bis 1, insbesondere von 4 bis 2, vorzugsweise von 3,5 bis 2,5, gemäß Snyder auf. Ein Lösungsmittelgemisch aus 60 % Methanol/40 % Dichlormethan hat beispielsweise einen Polaritätsindex von 3,1 nach Snyder. Als unpolare Lösungsmittel kommen demnach beispielsweise halogenhaltige Lösungsmittel wie Chloroform, Dichlormethan oder Tetrachlorkohlenstoff in Frage. Ferner sind aliphatische Lösungsmittel wie Pentan, Hexan, Heptan oder Cyclohexan zu nennen. Daneben sind als unpolare Lösungsmittel aromatische Lösungsmittel wie Toluol oder Benzol zu nennen. Weiterhin kommen Ether wie Diethylether, tert.-Butylmethylether oder Tetrahydrofuran in Frage.

Die Lösungsmittel werden in Form von Lösungsmittelgemischen eingesetzt, welche polare organische Lösungsmittel und unpolarische organische Lösungsmittel umfassen.

Bevorzugterweise werden die polaren organischen und unpolaren organischen Lösungsmittel in einem Verhältnis von 1:1, insbesondere in einem Verhältnis von 1:2, vorzugsweise in einem Verhältnis von 1:10 (polar:unpolar), eingesetzt.

Diese Maßnahme hat den Vorteil, dass je nach Beschaffenheit der biologischen Materialien Lösungsmittelgemische angepaßt hergestellt werden können. Auf diese Weise können eine Vielzahl von Trennproblemen bearbeitet werden.

In einer weiteren bevorzugten Ausführungsform des Verfahrens werden ferner Tenside, insbesondere nichtionische Tenside, eingesetzt.

In einer weiteren bevorzugten Ausführungsform des Verfahrens werden als Tenside Copolymere, insbesondere Copolymere aus Polyethylenoxiden und Polypropylenoxiden, eingesetzt.

Vorteilhafterweise wurde gefunden, dass die Viskositatszunahme durch Zusatz von oberflächenaktiven Substanzen, den sogenannten Tensiden, verzögert wird. Als Folge der Verzögerung der Viskositatszunahme ergibt sich eine längere Interaktion zwischen den organischen Lösungsmitteln und den gelierenden biologischen Materialien. Die Extraktion der Inhaltsstoffe wird verbessert, insbesondere die Extraktion der lipophilen Inhaltsstoffe. Möglicherweise beruht die verbesserte Extraktion der Inhaltsstoffe, insbesondere der lipophilen Inhaltsstoffe, im Falle von Blut auf einer Verdrängung der Inhaltsstoffe aus Zellmembranen, insbesondere bei Lipiden auf einer Verdrängung aus Proteinbindungen. Vorteilhafterweise führt der Einsatz der Tenside zu einer Verbesserung von spektroskopischen Eigenschaften der Inhaltsstoffe der zu untersuchenden Überstände, vorzugsweise zu einer verstärkten Absorption der Inhaltsstoffe.

Bevorzugterweise werden solche Tenside verwendet, welche in dem Lösungsmittelgemisch löslich, frei von toxischen Eigenschaften sind und/oder die Analytik der im Überstand befindlichen Inhaltsstoffe unbeeinflußt lassen. Vorzugsweise bleiben Absorption und/oder Fluoreszenz der Inhaltsstoffe unverschlechtert meßbar.

Bevorzugterweise werden die Tenside in Konzentrationen eingesetzt, welche die Hämolyse von Blut ausschließen. Vorteilhafterweise werden als Tenside Copolymere, insbesondere Copolymere aus Polyethylenoxiden und Polypropylenoxiden, eingesetzt. Als Beispiel für CopolymerTenside kommen handelsübliche Pluronic^{®}-Tenside in Frage, vorzugsweise Pluronic^{®} 101.

In einer bevorzugten Ausführungsform werden die biologischen Materialien mit den organischen Lösungsmittelgemischen in einem Verhältnis von 1:50, insbesondere in einem Verhältnis von 1:10, vorzugsweise in einem Verhältnis von 1:3, behandelt.

Je nach Beschaffenheit der biologischen Materialien und Extraktionsvermögen der organischen Lösungsmittel kann es zweckmäßig sein, ein größeres oder kleineres Verhältnis von biologischen Materialien zu organischen Lösungsmitteln zu wählen, insbesondere hängt dies von der nachfolgenden Analytik ab. Vorzugsweise wird das Verhältnis so gewählt, dass Nachweis- bzw. Bestimmungsgrenze in der Analytik der Inhaltsstoffe berücksichtigt werden.

In einer weiteren Ausführungsform wird das Verfahren bei einer Temperatur im Bereich von 5 °C bis 60 °C, insbesondere im Bereich von 10 °C bis 40 °C, durchgeführt.

In einer weiteren Ausführungsform wird das Verfahren bei einem Druck zwischen 0,5 bar bis 5 bar, insbesondere zwischen 0,8 bar bis 2 bar, durchgeführt.

Theoretisch läßt sich das Verfahren in Temperatur- und/oder Druckbereichen durchführen, bei welchen eine Gelbildung zu erwarten ist. Daneben sind Lösungsmitteleigenschaften, insbesondere Schmelzpunkt, Siedepunkt, Flammpunkt, zu berücksichtigen. Erfindungsgemäß wird das Verfahren bei Raumtemperatur und Normaldruck durchgeführt.

In einer weiteren bevorzugten Ausführungsform des Verfahrens werden die biologischen Materialien zur Extraktion der Inhaltsstoffe während eines Zeitraumes von 10 Sekunden bis 10 Minuten, insbesondere von 10 Sekunden bis 5 Minuten, vorzugsweise von 10 Sekunden bis 3 Minuten, behandelt.

Vorteilhafterweise erfolgt die Verfestigung der biologischen Materialien erfindungsgemäß parallel zur Extraktion der Inhaltsstoffe. Der Zeitraum der Verfestigung läßt sich durch die Behandlung der biologischen Materialien mit den Lösungsmittelgemischen in gewünschter Weise steuern. Der Zusatz von Tensiden verlängert die Extraktionsdauer. Überraschenderweise wurde gefunden, dass die biologischen Materialien, insbesondere Blut, vorzugsweise Vollblut, innerhalb weniger Minuten durch die Behandlung gemäß dem erfindungsgemäßen Verfahren verfestigt werden. Rein aufgrund von Gravitationskräften wird durch zu Boden sinken der biologischen Materialien ein verfestigtes Sediment und ein darüber befindlicher Überstand gebildet. Der Überstand läßt sich bei Bedarf einfach entnehmen. Im Überstand gelöste Inhaltsstoffe der biologischen Materialien werden der eingehenderen Analytik zugeführt. Eine Abtrennung der biologischen Materialien vom Extrakt durch Zentrifugation und/oder Filtration erübrigt sich. Vorteilhafterweise ergibt sich somit durch das erfindungsgemäß durchgeführte Verfahren ein Zeitgewinn in der Analytik.

Die biologischen Materialien werden vor der Behandlung mit den organischen Lösungsmittelgemischen in eine lösungsmittelbeständige Umgebung überführt.

Als lösungsmittelbeständige Umgebung wird ein Gefäß mit einer hydrophoben Oberfläche, insbesondere ein Gefäß mit einer durch Silanisierung hydrophobierten Oberfläche, verwendet.

Beschrieben ist auch ein Gefäß mit einer hydrophoben Oberfläche, insbesondere ein Kunststoffgefäß, vorzugsweise aus Polypropylen.

Vorteilhafterweise können im erfindungsgemäßen Verfahren Gefäße mit hydrophoben Oberflächen verwendet werden. Die hydrophoben Oberflächen lassen sich im Falle von Glasgefäßen durch die Silanisierung der Glasoberfläche oder durch ein Ätzen mit Fluorwasserstoff herstellen. Beschrieben sind auch Kunststoffgefäße, vorzugsweise aus Polypropylen und der Einsatz von Verbundwerkstoffen für die Gefäße, insbesondere kunststoffbeschichtete Gefäße Erfindungsgemäß. Erfindungsgemäß werden Gefäße verwendet, welche so beschaffen sind, dass sie für spektroskopische Untersuchungen geeignet sind.

In einer weiteren bevorzugten Ausführungsform des Verfahrens werden die Inhaltsstoffe vor der Extraktion in eine lipophile Form überführt und/oder lipophil modifiziert.

Bevorzugterweise wird der Überstand auf lipophile Inhaltsstoffe untersucht. Die lipophilen Inhaltsstoffe können in den biologischen Materialien in gebundener Form, insbesondere in physikalisch und/oder chemisch gebundener Form, vorliegen. Daneben kann es von Interesse sein, Inhaltsstoffe lipophober bzw. hydrophiler Natur zu untersuchen.

Vorteilhafterweise wird die Lipophilisierung der Inhaltsstoffe auf chemischem und/oder physikalischem Weg erreicht. Als chemisch herbeigeführte Lipophilisierung sind Enzym-Substrat-Reaktionen zu nennen, die zu definierten Produkten führen. Beispielsweise wäre zu nennen eine Spaltung von Fettsäureestern mittels Lipasen oder die Umsetzung von lipophil modifizierten Substraten durch andere Enzyme. So ist zum Beispiel ein mit einem Farbstoffmolekül, wie DABCYL-NHS Ester, gekoppeltes Stärkemolekül trotzt des lipophilen Farbstoffs immer noch wasserlöslich. Mit der durch die Amylase verursachten Verdauung wird das Stärkemakromolekül schrittweise in kleinere Einheiten zerlegt. Dadurch wird der hydrophile Teil immer kleiner und das Gesamtmolekül immer lipophiler, bis das Farbstoffmolekül die Lösungseigenschaften dominiert und in die organische Phase übergeht. Als physikalisch herbeigeführte Lipophilisierung kommt ein Binden an lipophile Substanzen in Frage. Beispielsweise können hydrophile Substanzen an Antikörper binden, welche eine Lipophilie durch ihre lipophilen Teile für einen solchen Gesamtkomplex bewirken. Ferner können als lipophile Moleküle auch Chelatoren eingesetzt werden. Beispielsweise geht Eisen aus dem Blut durch die Bindung an einen lipophilen Chelator in die organische Phase über und kann dort analysiert werden.

In einer weiteren Ausführungsform des Verfahrens handelt es sich bei mindestens einem der Inhaltsstoffe um Kohlenhydrate, Lipide, Nukleinsäuren, Proteine, Mengenelemente, Spurenelemente und/oder Vitamine. Als Kohlenhydrate kommen Saccharide, insbesondere Polysaccharide, vorzugsweise Monosaccharide, in Frage. Als Lipide kommen Fettsäuren, Triglyceride (Triacylglyceride, Fettsäureester von Glycerin), Glycerophospholipide, wie Phosphatidsäuren, Lecithine, Cardiolipine, Plasmalogene, usw.; Sphingolipide, wie Sphingosine, Ceramide, Sphingophospho- und Sphingoglycolipide; Isoprenoide (Terpene), wie Steroide, insbesondere Steroidderivate, Carotinoide, usw.; Wachse (Ester langkettiger aliphatischer Alkohole mit Fettsäuren), und Lipopolysaccharide (Bestandteile der Zelloberfläche Gram-negativer Bakterien) in Frage. Als Vitamine kommen beispielsweise lipophile Vitamine in Frage, insbesondere Vitamin A, Vitamin D, Vitamin E und/oder Vitamin K. Daneben kommen als Inhaltsstoffe auch Provitamine, Profaktoren und/oder Retinoide (Vitamin-A-Derivate) in Frage. Vorzugsweise handelt es sich bei den Lipiden um Triglyceride, Fettsäuren, Cholesterine und/oder Carotine, insbesondere um Beta-Carotin. Als Inhaltsstoffe, welche in mehreren Substanzklassen vorkommen, sind insbesondere Hormone zu nennen.

In einer weiteren bevorzugten Ausführungsform des Verfahrens handelt es sich bei den Inhaltsstoffen um sekundäre Pflanzenstoffe. Als sekundäre Pflanzenstoffe kommen Alkaloide, Flavonoide und/oder Carotinoide, insbesondere Xantophylle und/oder Carotine, vorzugsweise Beta-Carotine, in Frage.

Sämtliche mit dem erfindungsgemäßen Verfahren zugängliche Inhaltsstoffe der biologischen Materialien können einer anschließenden Analytik zugeführt werden. Insbesondere umfaßt dies Derivate der oben angeführten Inhaltsstoffe.

In der erfindungsgemässen Ausführungsform des Verfahrens werden die Inhaltsstoffe spektro photo metrisch oder fluorimetrisch untersucht.

Beschrieben für die nachfolgende Analyse der Inhaltsstoffe sind sämtliche bekannte oder auch bisher unbekannte Analyse-Techniken. Eine Trennung der Inhaltsstoffe, beispielsweise durch chromatographische Verfahren, insbesondere mit Hochleistungs-Flüssigkeitschromatographie (high performance liquid chromatography, HPLC), kann sich für die weitere Analyse als förderlich erweisen. Erfindungsgemäß wird der Überstand analytischen Verfahren zugeführt, welche die Inhaltsstoffe spektro photo metrisch oder fluorimetrisch untersuchen. Als spektrometrische Verfahren beschrieben sind solche, welche die Inhaltsstoffe durch eine Wechselwirkung mit elektromagnetischer Strahlung untersuchen, beispielsweise NMR-, IR-, UV-Vis-, Laser-Raman-Spektroskopie. Daneben können sämtliche bekannte massenspektrometische Verfahren zur Anwendung kommen.

Beschrieben wird die Analyse kann zur Diagnose und/oder Therapieverfolgung von Erkrankungen, insbesondere von Stoffwechselerkrankungen und/oder Mangelerkrankungen.

Stoffwechselerkrankungen und/oder Mangelerkrankungen führen im Allgemeinen zu einem Überschuß oder einem Mangel, insbesondere einem Fehlen, von Substanzen im menschlichen und/oder tierischen Organismus. Daraus können Erkrankungen resultieren. Die vorliegende Erfindung ist geeignet für die Diagnose solcher Erkrankungen ist geeignet. Eine Therapieverfolgung, insbesondere zur Analyse von zur Therapie verwendeten pharmakologischen Wirkstoffen und/oder Nährstoffen, vorzugsweise zur Verfolgung einer Supplementierung von Nährstoffen, ist mit der vorliegenden Erfindung prinzipiell durchführbar.

Bevorzugterweise handelt es sich bei den Inhaltsstoffen im wesentlichen um lipophile Stoffe.

Die Messung kann mit einem Spektrophotometer, insbesondere einem Handphotometer, zur Messung von Inhaltsstoffen von biologischen Materialien in einem zylindrischen Gefäß erfolgen, wobei der Strahlengang des Photometers so eingestellt ist, dass die Messung eine obere Hälfte des Gefäßes erfaßt.

Unter einem zylindrischen Gefäß wird ein Gefäß verstanden, welches über seine gesamte Länge ein gleichbleibendes Profil aufweist. Als gleichbleibendes Profil kommt ein rechteckiger Querschnitt, insbesondere ein quadratischer Querschnitt, wie er in spektroskopischen Küvetten Verwendung findet, in Frage. Erfindungsgemäß finden für die Messung der Inhaltsstoffe der biologischen Materialien die Gefäße Verwendung, in welchen das erfindungsgemäße Verfahren durchgeführt wurde. Eserfolgt die Analyse der Inhaltsstoffe nach Extraktion ohne Zeitverzögerung im Spektrophotometer.

Zusammenfassend ist zu sagen, dass die hier vorliegende Erfindung die Extraktion von Inhaltsstoffen aus biologischen Materialien ermöglicht sowie weitere Analytik der Inhaltsstoffe vereinfacht. Insbesondere die Analytik von Blut wird hierdurch vereinfacht. Es kommt zu kaum messbaren Temperaturschwankungen bei der Durchführung des erfindungsgemäßen Verfahrens, eine Hämolyse von Blut wurde nicht beobachtet. Ferner werden aufwendige Trennschritte, wie die Filtration und/oder die Zentrifugation der Proben, nicht benötigt. Die Erfindung lässt sich in einem Schritt durchführen. Sie ist daher für die miniaturisierten Verfahren, insbesondere Hochdurchsatzverfahren, die in Form von Chiplaboren bekannt sind und vorzugsweise in den sogenannten "point-of-care"-Systemen Verwendung finden, besonders geeignet.

Weitere Merkmale und Vorteile der Erfindung ergeben sich aus der nachfolgenden Beschreibung bevorzugter Ausführungsformen anhand von Beispielen in Verbindung mit den Unteransprüchen. Hierbei können die einzelnen Merkmale jeweils für sich allein oder zu mehreren in Kombination miteinander verwirklicht sein.

### Detaillierte Beschreibung der Ausführungsbeispiele

Die Erfindung wird nachfolgend anhand ausgewählter Ausführungsbeispiele im Zusammenhang mit den beiliegenden Zeichnungen näher beschrieben und erläutert. Es zeigen:
- Fig. 1: durch Hämolyse verunreinigte Spektren im ultraviolettsichtbaren Bereich (UV-Vis-Spektrum) von Beta-Carotin aus Rinderserum,
- Fig. 2: ein zur Figur 1 vergleichbares UV-Vis-Spektrum von Beta-Carotin aus Rinderserum, allerdings hämolysefrei,
- Fig. 3: ein HPLC-Chromatogramm nach einer erfindungsgemäßen Extraktion von Carotinoiden aus menschlichem Vollblut,
- Fig. 4: ein tabellarischer Vergleich einer erfindungsgemäßen Extraktion von Beta-Carotin unter Einfluss eines Tensidzusatzes,
- Fig. 5: Lösungsmittelgemische, welche zwei Lösungsmittel aufweisen und ihr Einfluss auf eine Wiederfindung I von Beta-Carotin, Retinol und Tocopherol,
- Fig. 6: Lösungsmittelgemische, welche drei Lösungsmittel aufweisen und ihr Einfluss auf die Wiederfindung II von Beta-Carotin, Retinol und Tocopherol,
- Fig. 7: ein Vergleich einer Serumextraktion und einer erfindungsgemäß durchgeführten Vollblutextraktion,
- Fig. 8: die Wiederfindung III verschiedener Inhaltsstoffe mit verschiedenen Lösungsmittelgemischen von Ethanol:Isopropanol in der Extraktion, und
- Fig. 9: eine Verfestigung von Blut, in einem Gefäß mit unhydrophobierter Oberfläche und Gefäßen mit hydrophobierten Oberflächen.

### Ausführungsbeispiele

### Extraktion und Bestimmung von Vitamin A, Vitamin E und Carotinoiden aus Vollblut

Das von Rind, Pferd, Hund, Katze, Fledermaus oder Mensch stammende Vollblut wurde zunächst mit Gerinnungshemmern ungerinnbar gemacht, vorzugsweise mit Ethylendiamintetraacetat (EDTA). Dann wurde eine Probe des Vollblutes in ein Kunststoffröhrchen (Polypropylen, PP zu Vergleichszwecken) oder oberflächenbehandeltes (silanisiertes) Glasröhrchen gegeben. Die Probe wurde dann mit einem Gemisch von organischen Lösungsmitteln versetzt. Es wurde ein Gemisch von polaren organischen und unpolaren organischen Lösungsmitteln (Ethanol/Isopropanol/Isooctan; 1:4:10) mittels einer Spritze oder Pipette zu dem in einem Gefäß befindlichen Blut zugegeben. Ein auf diese Weise erhaltenes Gemisch wurde intensiv vorsichtig für 10 Sekunden manuell geschüttelt. Die Blutprobe zeigte eine Zunahme ihrer Viskosität. Anschließend erfolgt eine Phasentrennung durch Sedimentation für 5 Minuten. Nach 2 bis 3 Minuten wurde erneut geschüttelt und stehengelassen. Es bildete sich eine gelartige Verfestigung der Blutprobe im unteren Teil des Röhrchens aus. Im oberen Teil des Röhrchens bildete sich als Überstand eine organische Phase aus. Die organische Phase enthielt im wesentlichen Isooctan und extrahierte Inhaltsstoffe aus dem Blut. Die organische Phase war durch extrahierte Carotinoide gelblich gefärbt. Je nach Konzentration der Carotinoide in der Probe fiel die Färbung intensiver aus. Die Überstände wurden entweder direkt im Spektralphotometer vermessen oder zu Vergleichszwecken zunächst abpipettiert, eingeengt, im geeigneten Lösungsmittel aufgenommen und dann beispielsweise mittels Hochleistungs-Flüssigkeitschromatographie (high performance liquid chromatography, HPLC) vermessen.

### Extraktion und Bestimmung von Vitamin A, Vitamin E und Carotinoiden aus Serum

Die Versuchsdurchführung wurde wie beschrieben beibehalten. Ebenfalls die Vermessung der Überstände.

### Extraktion und Bestimmung von Vitamin A, Vitamin E und Carotinoiden aus Kolostralmilch

Als biologisches Material wurde Kollostralmilch von Rind oder Mensch verwendet. Versuchsdurchführung und Vermessung wurden wie angegeben vorgenommen.

### Extraktion und Bestimmung von Vitamin A, Vitamin E und Carotinoiden aus Lebergewebe

Lebergewebe wurde nach bekannten Verfahren in Puffern mittels Ultratorax-Behandlung homogenisiert.

Als biologisches Material wurde das flüssige bis zähflüssige Homogenisat eingesetzt, Versuchsdurchführung und Vermessung wurden wie angegeben vorgenommen.

### Extraktion und Nachweis von Cholesterin aus Vollblut, Serum, Kolostralmilch oder Lebergewebe

Wie in den vorangegangenen Beispielen beschrieben, wurden zunächst lipophile Inhaltsstoffe in den organischen Überstand extrahiert. Cholesterin wurde dann in einem Aliquot des organischen Extraktes mittels bekannter enzymatischer Verfahren bestimmt (zu Vergleichszwecken).

### Extraktion und Nachweis von nicht-veresterten Fettsäuren (NEFA) aus Vollblut, Serum oder Kolostralmilch

Wie in den vorangegangenen Beispielen beschrieben, wurden zunächst die lipophilen Inhaltsstoffe in den organischen Überstand extrahiert. Die NEFA wurden zunächst mittels Kupfer komplexiert, anschließend wurde ein solcher Fettsäure-Kupfer-Komplex durch eine Farbreaktion detektiert. Die Farbreaktion wurde im Spektralphotometer vermessen. Alle Schritte werden im Verfahren im gleichen Extraktionsröhrchen durchgeführt. Als Kupferreagenz wurde ein Gemisch aus Kupfer-Triethanolamin-Lösung 1 M TEA, 1 N Essigsäure, 6,45 % Cu(NO₃)₂; 9:1:10 v/v) verwendet. Das Kupferreagenz wird zum organischen Überstand zugegeben, nachdem sich eine deutliche Verfestigung des Vollblutes eingestellt hat. Nach intensivem Schütteln innerhalb von 20 Sekunden und einer Inkubationszeit von etwa 30 Minuten wurde dem Überstand ein Farbreagenz (0,5 % Lösung aus Diphenylcarbazon und Diphenylcarbazid (5:95 %) oder eine 0,5 %ige Lösung aus Diphenylcarbazon in Methanol) zugegeben und geschüttelt. Die Farbveränderung wurde im Spektralphotometer bei 550 nm gemessen.

### Nachweis von Lipaseaktivität

Zum Nachweis der Lipaseaktivität im Vollblut oder im Serum wurden Vollblut-Proben oder Serum-Proben zunächst mit Substrat versorgt. Als Substrat wurde ein Fettsäureester mit einer fluoreszierenden Fettsäure verwendet, welcher in Gallensäure emulgiert war. In der Probe vorhandene Lipase spaltet das Substrat. Nach einer Inkubation der Probe wird diese mit einem der beschriebenen Lösungsmittelgemische extrahiert und die freigesetzten markierten Fettsäuren vermessen. Die Signalintensität ist proportional zur Konzentration des Enzyms im Blut.

### Figurenbeschreibung

### Figur 1

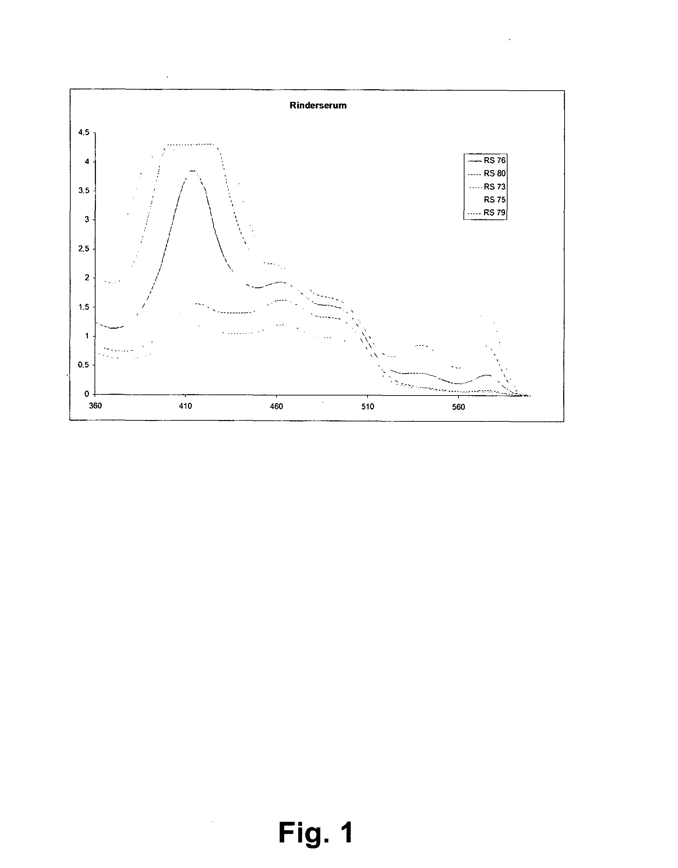

Figur 1 zeigt ein Diagramm, in welchem fünf verschiedene UV-Vis-Spektren von Beta-Carotin-Extrakten aus Rinderserumproben (RS73, RS75, RS76, RS79 RS80) im Wellenlängenbereich von 360 nm bis 580 nm mit ihren Absorptionen dargestellt sind. Aus dem Stand der Technik bekannte Verfahren wurden verwendet, um Blutzellen aus den Blutproben zu entfernen und Blutseren zu erhalten. In den Blutseren wurde Beta-Carotin direkt vermessen. In solchen Extrakten werden charakteristische Banden für Beta-Carotin üblicherweise bei 450 nm und 480 nm neben einer leichten Schulter bei 425 nm erwartet. Sämtliche Proben lieferten durch Hämolyse von Erythrozyten beeinträchtigte Spektren. Es wurde eine Verschiebung der charakteristischen Banden zu größeren Wellenlängen festgestellt. Drei der Proben waren durch die Hämolyse so stark verunreinigt, dass eine Auswertung aufgrund von starker Serumfärbungen unmöglich wurde.

### Figur 2

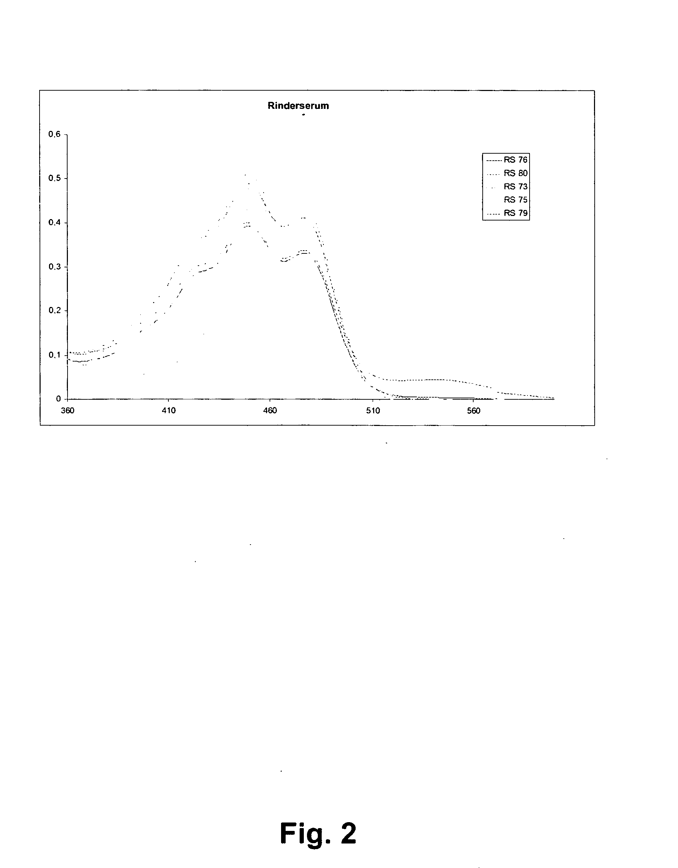

Figur 2 zeigt weitere fünf UV-Vis-Spektren von Beta-Carotin. Das Beta-Carotin wurde aus dem in Fig. 1 beschriebenen Rinderblut gewonnen, jedoch wurde die Extraktion aus Vollblut nach einem erfindungsgemäßen Verfahren durchgeführt. In allen Proben war nur noch das für Beta-Carotin typische Spektrum zu sehen.

### Figur 3

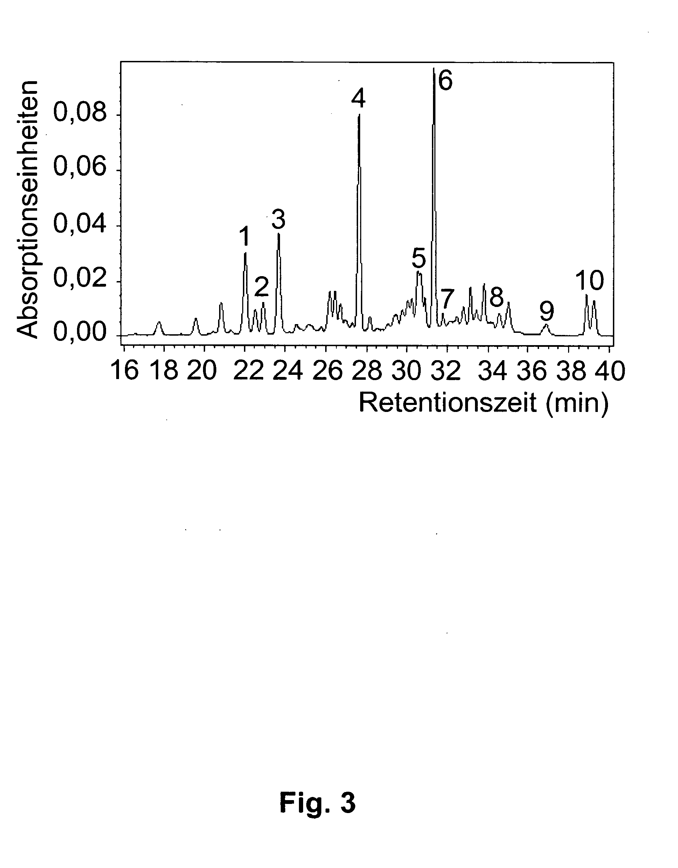

Figur 3 zeigt zu Vergleichszwecken ein HPLC-Chromatogramm eines gewonnenen Extraktes. Der Extrakt von Carotinoiden wurde aus menschlichem Vollblut gewonnen. Absorptionseinheiten wurden gegen die Retentionszeit in Minuten aufgetragen. Signale, sogenannte "Peaks", wurden von 1 = Lutein, 2 = Zeaxanthin, 3 = Canthaxanthin, 4 = Beta-Cryptoxanthin, 5 = Alpha-Carotin und 13-cis-Beta-Carotin, 6 = Beta-Carotin, 7 = 9-cis-Beta-Carotin, 8 und 9 = isomere cis-Formen von Lycopin und von 10 = trans-Lycopin erhalten. Die Signale sind nahezu symmetrisch, d.h. nahezu frei von sogenanntem Signalnachlauf ("Peak-Tailing"), und in einigen Fällen basisliniengetrennt.

### Figur 4

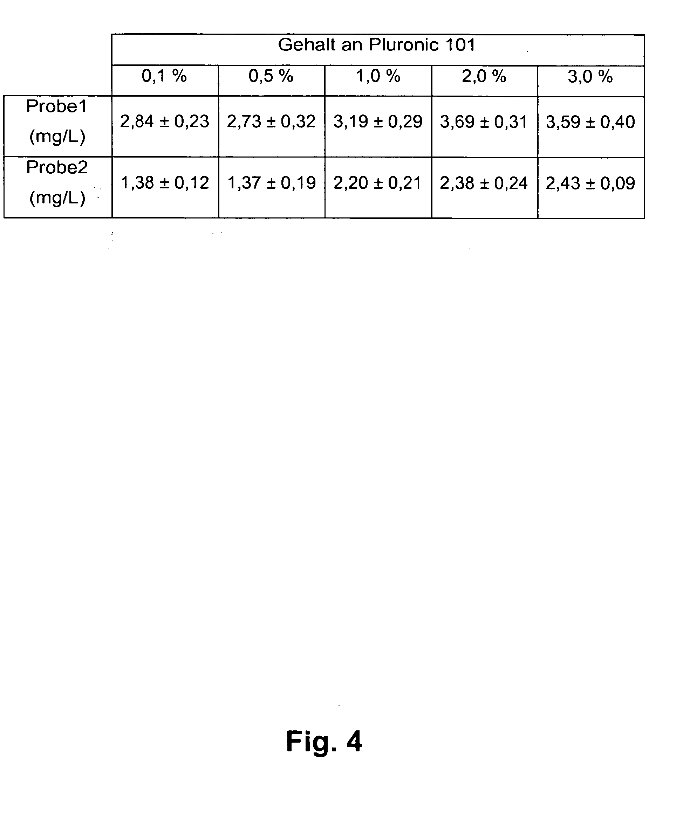

Figur 4 zeigt eine Tabelle, die einen Verlauf von Beta-Carotin-Konzentrationen abhängig von verschiedenen Zusätzen eines Tensids angibt. Als Tensid wurde Pluronic 101 in verschiedenen Konzentrationen (0,1% - 3,0%) eingesetzt. Das Tensid wurde in den angegebenen Konzentrationen zu Aliquoten von zwei unterschiedlichen Rinderblutproben gegeben. Die Absorptionen von Beta-Carotin wurden spektrophotometrisch bestimmt, Konzentrationen von Beta-Carotin sind als Mittelwert ± Standardabweichung in mg/L berechnet. Eine Erhöhung der Tensidkonzentration führte in beiden Blutproben zu einer Erhöhung der Beta-Carotin-Konzentrationen in den Extrakten.

### Figur 5

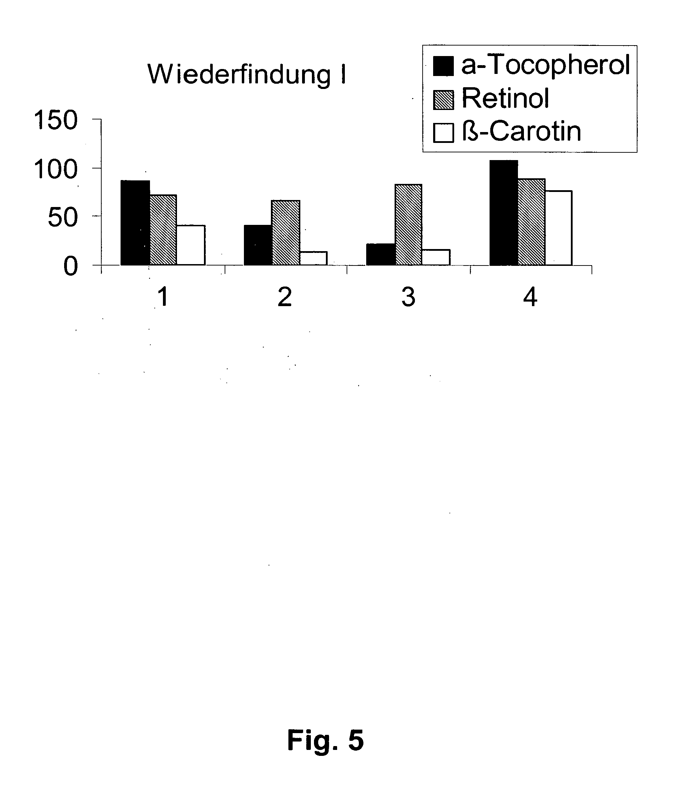

Figur 5 zeigt Ergebnisse von Experimenten zur Wiederfindung von Alpha-Tocopherol (Vitamin E), Retinol (Vitamin A) und Beta-Carotin. Rindervollblut wurde in vier Teile aliquotiert und nach dem erfindungsgemäßen Verfahren extrahiert. Jeder Aliquot betrug 250 µL. Es wurden folgende Lösungsmittelgemische im angegebenen Volumenverhältnis zur Extraktion verwendet: 1 = Ethanol/Isooctan (1:2 VN), 2 = Methanol/Isooctan (1:2 VN), 3 = Essigsäureethylester/Isooctan (1:2 V/V), 4 = Isopropanol/Isooctan (1:2 V/V). HPLC wurde zu Vergleichszwecken als Analytik verwendet. Retinol wurde als Analyt mit einer Wiederfindung von minimal 65 % im Methanol/Isooctan-Gemisch und von maximal 88 % im Isopropanol/Isooctan-Gemisch bestimmt. Alpha-Tocopherol wurde mit minimal 21 % im Essigsäureethylester/Isooctan-Gemisch und mit maximal 107 % Isopropanol/Isooctan-Gemisch wiedergefunden. Eine im Vergleich schlechtere Wiederfindung zu den anderen Analyten wurde in den Lösungsmittelgemischen 1 bis 3 für Beta-Carotin mit minimal 13 % und maximal 40 % bestimmt. Eine optimale Wiederfindung wurde für alle Analyten im Isopropanol/Isooctan-Gemisch ermittelt.

### Figur 6

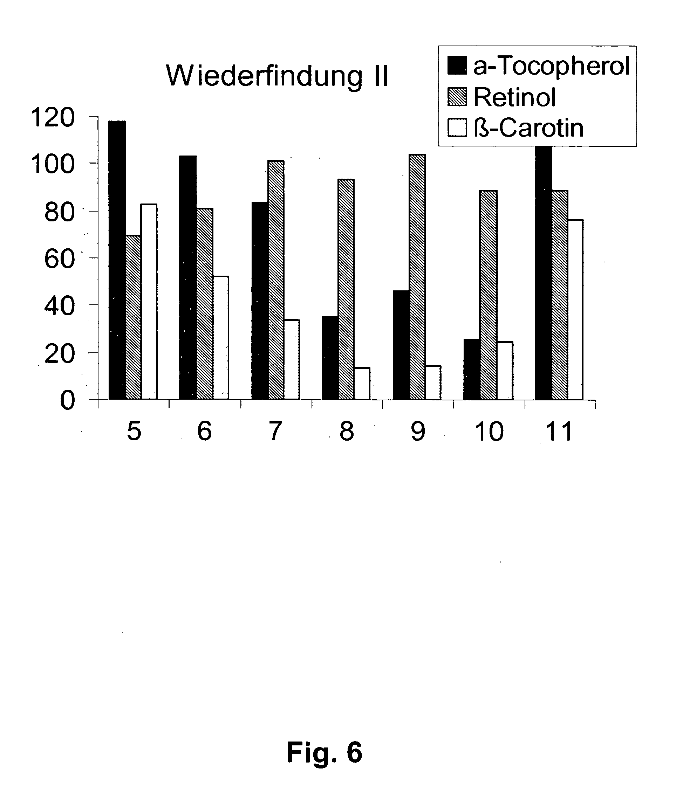

Figur 6 zeigt Ergebnisse von Experimenten (zu Vergleichszwecken) zur Wiederfindung von Alpha-Tocopherol (Vitamin E), Retinol (Vitamin A) und Beta-Carotin. Die Wiederfindung wurde, wie in der Beschreibung zu Figur 5 angegeben, durchgeführt. Es wurden die Lösungsmittelgemische 5 bis 11 verwendet: 5 = Ethanol/Isopropanol/Isooctan (1:1:4; V/V), 6 = Methanol/Isopropanol/Isooctan (1:1:4; VN), 7 = Essigsäureethylester/Isopropanol/Isooctan (1:1:4; VN), 8 = Wasser/Isopropanol/Isooctan (1:1:4; VN), 9 = Natriumchloridlösung (0,9%)/Ethanol/Isopropanol/Isooctan (1:1:4; VN), 10 = n-Butanol/Isopropanol/Isooctan (1:1:4; VN), 11 = Isopropanol/Isooctan (2:4; VN). Die Lösungsmittelgemische 5 bis 10 wurden im Vergleich zum Lösungsmittelgemisch 11, das Lösungsmittelgemisch 4 aus Figur 5 entspricht, hier allerdings in doppelter Menge, eingesetzt. Es wurden zusätzlich verschiedene protische Lösungsmittel, wie Alkohole, Wasser, Kochsalzlösung, zum "Basis-Lösungsmittelgemisch Isopropanol/Isooctan gegeben. Es zeigte sich, dass Alpha-Tocopherol und Beta-Carotin beide vergleichbare Abnahmen und Zunahmen in ihrer Wiederfindung in den Lösungsmittelgemischen 5 bis 10 aufwiesen. Neben dem für alle Analyten optimalen Lösungsmittelgemisch Isopropanol/Isooctan zeigte auch das Ethanol/Isopropanol/Isooctan-Gemisch hohe Wiederfindungen für alle Analyten.

### Figur 7

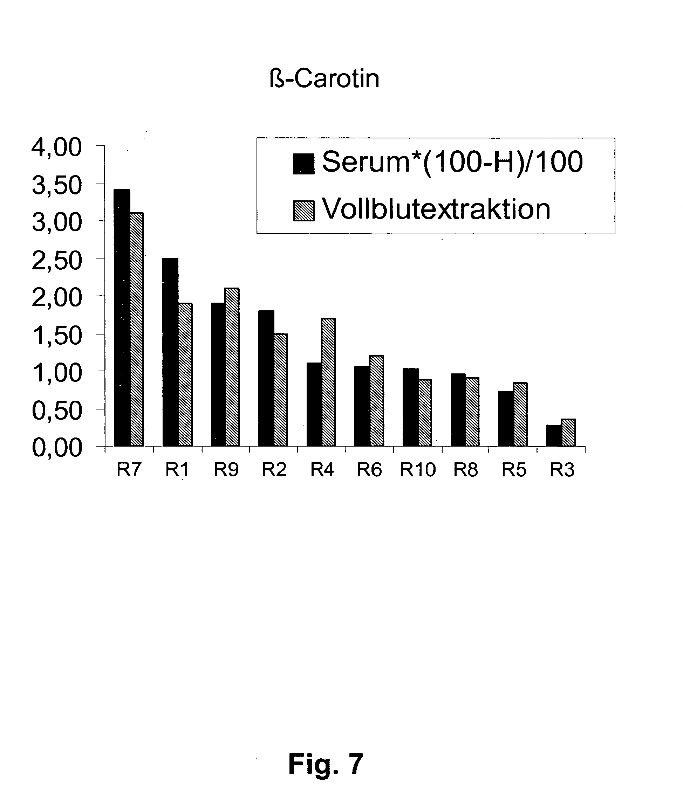

Fig. 7 zeigt einen Vergleich von Extraktionsergebnissen zwischen der klassischen Extraktion von Serum mit der Extraktion nach dem erfindungsgemäßen Verfahren aus Vollblut. Als Analyt wurde Beta-Carotin in 10 verschiedenen Blutproben von Kühen untersucht. Die Wiederfindung des Analyten im erfindungsgemäßen Verfahren ist bei sämtlichen Blutproben nahezu identisch zum klassischen Verfahren.

### Figur 8

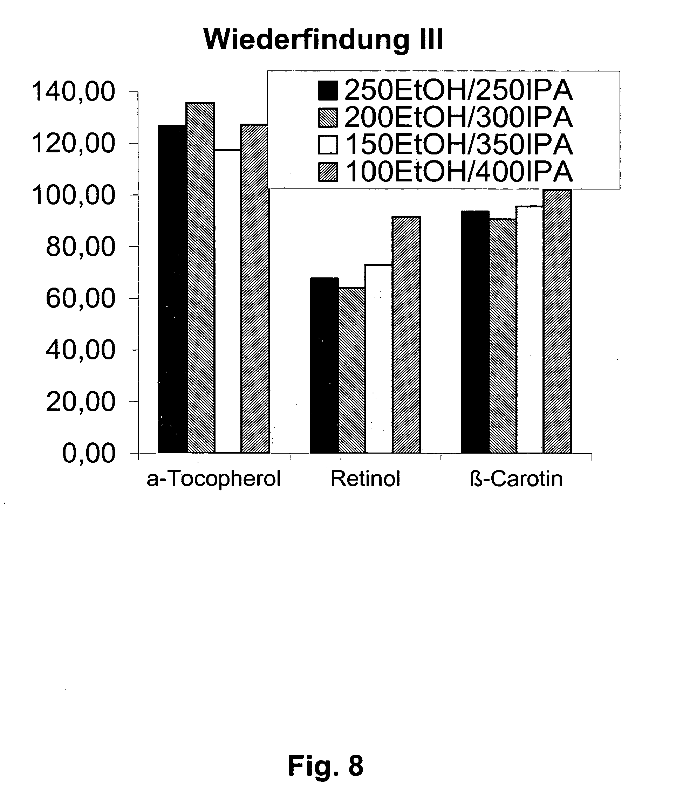

Fig. 8 untersucht einen Einfluss des Lösungsmittel-Verhältnisses von Ethanol zu Isopropanol im Lösungsmittelgemisch Ethanol/Isopropanol/Isooctan auf die Extraktion der Analyten Alpha-Tocopherol (Vitamin E), Retinol (Vitamin A) und Beta-Carotin. Legenden-Angaben beziehen sich auf einen Anteil des jeweiligen polaren Lösungsmittels in µL. Als Anteil des unpolaren Lösungsmittels Isooctan wurde 1 mL verwendet. Als Vollblutprobe vom Rind wurden 250 µL eingesetzt.

### Figur 9

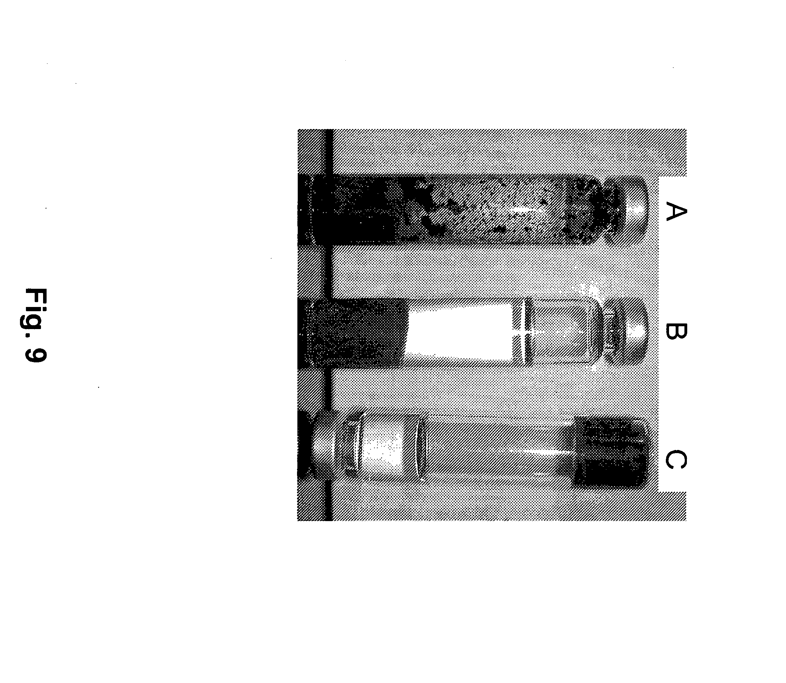

Fig. 9 zeigt einen Einfluss von Gefäßoberflächen auf eine Adhäsion der Blutzellen und das Ergebnis der Verfestigung. In Figur 9A ist die Extraktion in einem Gefäß mit unbehandelter Oberfläche, in Figur 9B in einem Gefäß mit durch Silanisierung hydrophobierter Oberfläche, gezeigt. In Figur 9C ist die vollständige Verfestigung der Blutzellen nach ca 10 Minuten durch Umdrehen des Röhrchens demonstriert.

Die Wiederfindung für die einzelnen Analyten lag im Durchschnitt bei Blutproben von Kühen und Pferden bei 106 % für Alpha-Tocopherol (Vitamin E), 89% für Retinol (Vitamin A) und 95 % für Beta-Carotin.

## Patentansprüche

1. Verfahren zur Analyse von Inhaltsstoffen, insbesondere von Lipiden und/oder Vitaminen, biologischer Materialien mit einer Anreicherung der Inhaltsstoffe aus den biologischen Materialien, bei welchem den biologischen Materialien, welche sich in einem für spektroskopische Untersuchungen geeigneten Gefäß mit einer hydrophoben Oberfläche befinden in einem einzigen Schritt ein Lösungsmittelgemisch zugegeben wird, welches polare organische Lösungsmittel und unpolare organische Lösungsmittel umfasst, welches die Inhaltsstoffe extrahiert, wobei die biologischen Materialien bei der Extraktion in eine verfestigte Form überführt werden, wobei eine Verfestigung eine Viskositätszunahme der biologischen Materialien bedeutet, so dass ein verfestigtes Sediment und eine flüssige organische Phase als Überstand rein aufgrund von Gravitationskräften durch zu Boden sinken der biologischen Materialien ohne Zentrifugation gebildet werden, und die in den Überstand extrahierten Inhaltsstoffe im selben Gefäß direkt spektrophotometrisch oder fluorimetrisch untersucht werden.

2. Verfahren nach Anspruch 1, **dadurch gekennzeichnet, dass** die biologischen Materialien pflanzliche, tierische, menschliche und/oder mikrobielle Materialien umfassen, welche insbesondere aus Zellkulturen stammen.

3. Verfahren nach Anspruch 1 oder 2, **dadurch gekennzeichnet, dass** die biologischen Materialien vor ihrem Einsatz Vorbehandlungen, insbesondere Reinigungsvorgängen, unterworfen werden.

4. Verfahren nach einem der vorhergehenden Ansprüche, **dadurch gekennzeichnet, dass** als biologische Materialien Gewebe, Organe und/oder Körperflüssigkeiten verwendet werden.

5. Verfahren nach Anspruch 4, **dadurch gekennzeichnet, dass** als Körperflüssigkeiten Blut, Plasma, Serum, Harn, Amnionflüssigkeit, Uterusflüssigkeit, Follikelflüssigkeit, Synovia, Sperma, Lungenflüssigkeit und/oder Sekrete verwendet werden.

6. Verfahren nach einem der Ansprüche 1 bis 3, **dadurch gekennzeichnet, dass** als biologische Materialien Lebensmittel tierischen und/oder pflanzlichen Ursprungs, insbesondere Homogenisate von Lebensmitteln, eingesetzt werden.

7. Verfahren nach einem der vorhergehenden Ansprüche, **dadurch gekennzeichnet, dass** polar protische Lösungsmittel als polares organisches Lösungsmittel verwendet werden, insbesondere Alkohole.

8. Verfahren nach Anspruch 7, **dadurch gekennzeichnet, dass** die polar protischen Lösungsmittel ausgewählt sind aus der Gruppe, umfassend Methanol, Ethanol, 1-Propanol, 2-Propanol (Isopropanol), Butanol, Pentanol, Hexanol sowie Gemische davon.

9. Verfahren nach einem der Ansprüche 1 bis 6, **dadurch gekennzeichnet, dass** als unpolares organisches Lösungsmittel insbesondere Alkane, vorzugsweise C5- bis C12-Alkane, verwendet werden.

10. Verfahren nach Anspruch 9, **dadurch gekennzeichnet, dass** als unpolare organische Lösungsmittel Hexan, Heptan und/oder Octan, insbesondere Isooctan, vorzugsweise Gemische davon verwendet werden.

11. Verfahren nach einem der vorhergehenden Ansprüche, **dadurch gekennzeichnet, dass** ferner Tenside, insbesondere nichtionische Tenside, eingesetzt werden.

12. Verfahren nach Anspruch 11, **dadurch gekennzeichnet, dass** als Tenside Copolymere, insbesondere Copolymere aus Polyethylenoxiden und Polypropylenoxiden, eingesetzt werden.

13. Verfahren nach einem der vorhergehenden Ansprüche, **dadurch gekennzeichnet, dass** die biologischen Materialien mit dem Lösungsmittelgemisch in einem Verhältnis von 1:50, insbesondere in einem Verhältnis von 1:10, vorzugsweise in einem Verhältnis von 1:3, behandelt werden.

14. Verfahren nach einem der vorhergehenden Ansprüche, **dadurch gekennzeichnet, dass** das Verfahren bei einer Temperatur im Bereich von 5°C bis 60°C, insbesondere im Bereich von 10°C bis 40°C, durchgeführt wird.

15. Verfahren nach einem der vorhergehenden Ansprüche, **dadurch gekennzeichnet, dass** das Verfahren bei einem Druck zwischen 0,5 bar bis 5 bar, insbesondere zwischen 0,8 bar bis 2 bar, durchgeführt wird.

16. Verfahren nach einem der vorhergehenden Ansprüche, **dadurch gekennzeichnet, dass** die biologischen Materialien zur Extraktion der Inhaltsstoffe während eines Zeitraumes von 10 Sekunden bis 10 Minuten, insbesondere von 10 Sekunden bis 5 Minuten, vorzugsweise von 10 Sekunden bis 3 Minuten, behandelt werden.

17. Verfahren nach einem der vorhergehenden Ansprüche, **dadurch gekennzeichnet, dass** die Inhaltsstoffe vor der Extraktion in eine lipophile Form überführt und/oder lipophil modifiziert werden.

18. Verfahren nach Anspruch 17, **dadurch gekennzeichnet, dass** die wasserlöslichen Inhaltsstoffe vor der Extraktion mit einem lipophilen Molekül und/oder einem lipophil modifizierten Molekül einen lipophilen Komplex bilden.

19. Verfahren nach einem der vorhergehenden Ansprüche, **dadurch gekennzeichnet, dass** wasserlösliche Inhaltsstoffe durch Extraktion von lipophil oder lipophil modifizierten Produkten nachgewiesen werden, welche aus Enzym-Substrat-Reaktionen erhalten worden sind.

20. Verfahren nach einem der vorhergehenden Ansprüche, **dadurch gekennzeichnet, dass** es sich bei mindestens einem der Inhaltsstoffe um Kohlenhydrate, Lipide, Nukleinsäuren, Proteine, Mengenelemente, Spurenelemente und/oder Vitamine handelt.

21. Verfahren nach Anspruch 20, **dadurch gekennzeichnet, dass** es sich bei mindestens einem der Lipide um Triglyceride, Fettsäuren, Cholesterine und/oder Carotine handelt, insbesondere um Beta-Carotin.

22. Verfahren nach Anspruch 1, wobei ein Glassgefäß mit einer durch Silanisierung oder durch ein Ätzen mit Fluorwasserstoff hydrophobierten Oberfläche verwendet wird.

## Claims

1. A method for analysing ingredients, in particular lipids and/or vitamins, of biological materials with an enrichment of the ingredients from the biological materials, in which the biological materials, which are located in a vessel suitable for spectroscopic examinations with a hydrophobic surface, are mixed in a single step with a solvent mixture, which comprises polar organic solvents and nonpolar organic solvents, which extracts the ingredients, wherein the biological materials are converted during the extraction into a consolidated form, wherein a consolidation means an increase in viscosity of the biological materials so that a consolidated sediment and a liquid organic phase as a supernatant are formed, purely on the basis of gravitational forces since the biological materials sink to the bottom without centrifugation, and the ingredients extracted into the supernatant are examined directly in the same vessel by means of spectrophotometry or fluorometry.

2. The method according to Claim 1, **characterised in that** the biological materials comprise plant, animal, human and/or microbial materials, which are derived in particular from cell cultures.

3. The method according to Claim 1 or 2, **characterised in that** the biological materials are subjected before use thereof to pre-treatments, in particular purification processes.

4. The method according to one of the preceding claims, **characterised in that** tissues, organs and/or bodily fluids are used as biological materials.

5. The method according to Claim 4, **characterised in that** blood, plasma, serum, urine, amniotic fluid, uterine fluid, follicular fluid, synovia, sperm, pulmonary fluid and/or secretions are used as bodily fluids.

6. The method according to one of Claims 1 to 3, **characterised in that** food products of animal and/or plant origin, in particular homogenates of food products, are used as biological materials.

7. The method according to one of the preceding claims, **characterised in that** polarly protic solvents, in particular alcohols, are used as polar organic solvent.

8. The method according to Claim 7, **characterised in that** the polarly protic solvents are selected from the group comprising methanol, ethanol, 1-propanol, 2-propanol (isopropanol), butanol, pentanol, hexanol and mixtures thereof.

9. The method according to one of Claims 1 to 6, **characterised in that** alkanes in particular, preferably C5 to C12 alkanes, are used as nonpolar organic solvent.

10. The method according to Claim 9, **characterised in that** hexane, heptane and/or octane, in particular isooctane, preferably mixtures thereof, are used as nonpolar organic solvent.

11. The method according to one of the preceding claims, **characterised in that** surfactants, in particular nonionic surfactants, are also used.

12. The method according to Claim 11, **characterised in that** copolymers, in particular copolymers frompolyethylene oxides and polypropylene oxides, are used as surfactants.

13. The method according to one of the preceding claims, **characterised in that** the biological materials are treated with the solvent mixture in a ratio of 1:50, in particular in a ratio of 1:10, preferably in a ratio of 1:3.

14. The method according to one of the preceding claims, **characterised in that** the method is carried out at a temperature in the range from 5 °C to 60 °C, in particular in the range from 10 °C to 40 °C.

15. The method according to one of the preceding claims, **characterised in that** the method is carried out at a pressure between 0.5 bar to 5 bar, in particular between 0.8 bar to 2 bar.

16. The method according to one of the preceding claims, **characterised in that** the biological materials are treated for extraction of the ingredients for a period from 10 seconds to 10 minutes, in particular from 10 seconds to 5 minutes, preferably from 10 seconds to 3 minutes.

17. The method according to one of the preceding claims, **characterised in that**, before the extraction, the ingredients are converted into a lipophilic form and/or are lipophilically modified.

18. The method according to Claim 17, **characterised in that**, before the extraction, the water-soluble ingredients form a lipophilic complex with a lipophilic molecule and/or a lipophilically modified molecule.

19. The method according to one of the preceding claims, **characterised in that** water-soluble ingredients are detected by extracting lipophilic products or lipophilically modified products that have been obtained from enzyme-substrate reactions.

20. The method according to one of the preceding claims, **characterised in that** at least one of the ingredients is constituted by carbohydrates, lipids, nucleic acids, proteins, macroelements, trace elements and/or vitamins.

21. The method according to Claim 20, **characterised in that** at least one of the lipids is constituted by triglycerides, fatty acids, cholesterols and/or carotenes, in particular beta-carotene.

22. The method according to Claim 1, wherein a glass vessel with a surface made hydrophobic by silanisation or by etching with hydrogen fluoride is used.

## Revendications

1. Procédé permettant d'analyser des constituants, notamment des lipides et/ou des vitamines, de matières biologiques, comportant un enrichissement en lesdits constituants issus de matières biologiques et consistant à ajouter, en une seule étape, un mélange de solvants auxdites matières biologiques se trouvant dans un récipient lequel est adapté à des analyses spectroscopiques et lequel présente une surface hydrophobe, ledit mélange comprenant des solvants organiques polaires et des solvants organiques non-polaires et permettant d'extraire lesdits constituants, lesdites matières biologiques étant lors de cette extraction transformées de manière à adopter un état solidifié, ladite solidification se traduisant par une augmentation de la viscosité desdites matières biologiques, faisant en sorte qu'un sédiment solidifié et une phase organique liquide en tant que surnageant soient formés uniquement par l'action de la gravité précipitant lesdites matières biologiques vers le fond et sans centrifugation, et à procéder directement dans le même récipient à une analyse spectrophotométrique ou fluorimétrique desdits constituants se trouvant dans le surnageant suite à ladite extraction.

2. Procédé selon la revendication 1, **caractérisé en ce que** lesdites matières biologiques comprennent des matières végétales, animales, humaines et/ou microbiennes, issues notamment de cultures cellulaires.

3. Procédé selon les revendications 1 ou 2, **caractérisé en ce que** lesdites matières biologiques subissent des traitements préalables, notamment des processus de purification, avant d'être mises en oeuvre.

4. Procédé selon l'une des revendications précédentes, **caractérisé en ce que** l'on utilise en tant que matières biologiques des tissus, des organes et/ou des liquides biologiques.

5. Procédé selon la revendication 4, **caractérisé en ce que** l'on utilise en tant liquides biologiques du sang, du plasma, du sérum, de l'urine, du liquide amniotique, du liquide utérin, du liquide folliculaire, du liquide synovial, du sperme, du liquide issu des poumons et/ou des produits sécrétés.

6. Procédé selon l'une des revendications 1 à 3, **caractérisé en ce que** l'on met en oeuvre en tant que matières biologiques des aliments d'origine animale et/ou végétale, notamment des aliments homogénéisés.

7. Procédé selon l'une des revendications précédentes, **caractérisé en ce que** l'on utilise en tant que solvant organique polaire des solvants polaires protiques, notamment des alcools.

8. Procédé selon la revendication 7, **caractérisé en ce que** lesdits solvants polaires protiques sont choisis dans le groupe comprenant le méthanol, l'éthanol, le 1-propanol, le 2-propanol (isopropanol), le butanol, le pentanol, l'héxanol ainsi que leurs mélanges.

9. Procédé selon l'une des revendications 1 à 6, **caractérisé en ce que** l'on utilise en tant que solvant organique non-polaire notamment des alcanes, de préférence des alcanes en C5 à C12.

10. Procédé selon la revendication 9, **caractérisé en ce que** l'on utilise, en tant que solvants organiques non-polaires l'hexane, l'heptane et/ou l'octane, notamment l'isooctane, s'agissant préférentiellement de leurs mélanges.

11. Procédé selon l'une des revendications précédentes, **caractérisé en ce que** l'on met en oeuvre, en outre, des agents tensioactifs, notamment des agents tensioactifs non-ioniques.

12. Procédé selon la revendication 11, **caractérisé en ce que** l'on utilise en tant qu'agents tensioactifs des copolymères, notamment des copolymères à base de polyoxyéthylène et de polyoxypropylène.

13. Procédé selon l'une des revendications précédentes, **caractérisé en ce que** lesdites matières biologiques sont traitées avec le mélange de solvants dans un rapport de 1 : 50, notamment dans un rapport de 1 : 10, de préférence dans un rapport de 1 : 3.

14. Procédé selon l'une des revendications précédentes, **caractérisé en ce que** ledit procédé est mis en oeuvre à une température comprise entre 5 °C et 60 °C, notamment comprise entre 10 °C et 40 °C.

15. Procédé selon l'une des revendications précédentes, **caractérisé en ce que** ledit procédé est mis en oeuvre à une pression entre 0,5 bar et jusqu'à 5 bar, notamment entre 0,8 bar et jusqu'à 2 bar.

16. Procédé selon l'une des revendications précédentes, **caractérisé en ce que** lesdites matières biologiques sont traitées durant un laps de temps de 10 secondes à 10 minutes, notamment de 10 secondes à 5 minutes, préférentiellement de 10 secondes à 3 minutes, pour en extraire lesdits constituant.

17. Procédé selon l'une des revendications précédentes, **caractérisé en ce qu'**avant ladite extraction, lesdits constituants sont transformés de manière à adopter un état lipophile et/ou subissent une modification les rendant lipophiles.

18. Procédé selon la revendication 17, **caractérisé en ce qu'**avant ladite extraction, les constituants hydrosolubles forment un complexe lipophile avec une molécule lipophile et/ou une molécule rendue lipophile par modification.

19. Procédé selon l'une des revendications précédentes, **caractérisé en ce que** des constituants hydrosolubles sont détectés en extrayant des produits lipophiles ou rendus lipophiles par modification lesquels sont issus d'une réaction de type enzyme-substrat.

20. Procédé selon l'une des revendications précédentes, **caractérisé en ce qu'**au moins un desdits constituants est un glucide, un lipide, un acide nucléique, une protéine, un macroélément, un oligoélément et/ou une vitamine.

21. Procédé selon la revendication 20, **caractérisé en ce qu'**au moins un des lipides est un triglycéride, un acide gras, un cholestérol et/ou un carotène, notamment le bêta-carotène.

22. Procédé selon la revendication 1, le récipient en verre utilisé présentant une surface hydrophobée par silanisation ou par gravure chimique à l'aide de fluorure d'hydrogène.
